# EUROPEAN PATENT SPECIFICATION

(11) **EP 1 144 624 B1**
(45) Date of publication and mention of the grant of the patent: **04.05.2011**
(21) Application number: 00905230.9
(22) Date of filing: 11.02.2000
(51) Int. Cl.: C12N 15/12, C07K 14/705, G01N 33/50

(54) **HUMAN AND MOUSE TREK-1 POTASSIUM CHANNEL PROTEIN AND THEIR USE**
HUMANES UND MAUS TREK-1 KALIUMKANAL PROTEIN UND DESSEN VERWENDUNG
PROCEDE D'IDENTIFICATION DE SUBSTANCES ANESTHESIQUES

(30) Priority: 12.02.1999 US 119727 P; 11.02.2000 US 503089
(43) Date of publication of application: 17.10.2001
(73) Proprietor: CENTRE NATIONAL DE LA RECHERCHE SCIENTIFIQUE, 75016 Paris Cédex (FR)
(72) Inventor: LAZDUNSKI, Michel, F-06000 Nice (FR); HONORE, Eric, F-06160 Juan-les-Pins (FR); LESAGE, Florian, F-06000 Nice (FR); ROMEY, Georges, 61bis Corniche Fleurie, 06200 Nice (FR); PATEL, Amanda J., F-06160 Juan-les-Pins (FR)
(74) Representative: Novagraaf Technologies
(86) International application number: PCT/IB2000/000226
(87) International publication number: WO 2000/047738

(56) References cited:
- EP-A- 0 799 889
- PRICE ET AL.: "Homo sapiens two-pore potassium channel TPKC1 mRNA, complete cds" EMBL SEQUENCE DATABASE, 6 January 1999 (1999-01-06), XP002145556 HEIDELBERG DE
- FINK M ET AL: "Cloning, functional expression an brain localization of a novel unconventional outward rectifier K+ channel" EMBO JOURNAL,GB,OXFORD UNIVERSITY PRESS, SURREY, vol. 15, no. 24, 16 December 1996 (1996-12-16), pages 6854-6862, XP002085602 ISSN: 0261-4189 -& FINK ET AL.: "Mus musculus TREK-1 K+ channel subunit mRNA, complete cds" EMBL SEQUENCE DATABASE, 30 January 1997 (1997-01-30), XP002145557 HEIDELBERG DE -& FINK ET AL.: "Outward rectifying potassium channel protein TREK-1 (two-pore potassium channel TPKC1) (TREK-1 K+ channel subunit) " EMBL SEQUENCE DATABASE, 1 May 1997 (1997-05-01), XP002145558 HEIDELBERG DE
- CORREA A: "Gating kinetics of Shaker K+ channels are differentially modified by general anesthetics" AMERICAN JOURNAL OF PHYSIOLOGY CELL PHYSIOLOGY, vol. 44, 1998, pages C1009-C1021, XP002145559
- DUPRAT F ET AL: "TASK, A HUMAN BACKGROUND K+ CHANNEL TO SENSE EXTERNAL PH VARIATIONSNEAR PHYSIOLOGICAL PH" EMBO JOURNAL,GB,OXFORD UNIVERSITY PRESS, SURREY, vol. 16, no. 17, 1997, pages 5464-5471, XP000914717 ISSN: 0261-4189
- DATABASE BIOSIS [Online] BIOSCIENCES INFORMATION SERVICE, PHILADELPHIA, PA, US; 1998 LOPES C M B ET AL: "Actions of general anaesthetics and arachidonic acid pathway inhibitors on K+ currents activated by volatile anaesthetics and FMRFamide in molluscan neurones." Database accession no. PREV199800483606 XP002145561 & BRITISH JOURNAL OF PHARMACOLOGY, vol. 125, no. 2, 1998, pages 309-318, ISSN: 0007-1188
- DATABASE BIOSIS [Online] BIOSCIENCES INFORMATION SERVICE, PHILADELPHIA, PA, US; 1989 TAS P W L ET AL: "VOLATILE ANESTHETICS INHIBIT THE ION FLUX THROUGH CALCIUM-ACTIVATED POTASSIUM CHANNELS OF RAT GLIOMA C6 CELLS" Database accession no. PREV198988111203 XP002145562 & BIOCHIMICA ET BIOPHYSICA ACTA, vol. 983, no. 2, 1989, pages 264-268, ISSN: 0006-3002
- PATEL ET AL.: "Inhalational anesthetics activate two-pore-domain background K+ channels" NATURE NEUROSCIENCE, vol. 2, no. 5, May 1999 (1999-05), pages 422-426, XP002145560

## Description

### BACKGROUND OF THE INVENTION

### 1. Field of the Invention

The invention relates to a method for identifying substances that are capable of acting as anesthetics.

### 2. Background of the Related Art

Volatile anesthetics are a remarkable class of agents producing a safe, reversible state of unconsciousness with concurrent amnesia and analgesia. They have hyperpolarizing action on mammalian neurons. They activate an inhibitory synaptic K⁺ current (I_{K(As)} in molluscan pacemaker neurons which has been proposed to have an important role in general anaesthesia.

Volatile anesthetics hyperpolarize frog motor neurons, rat hippocampal neurons, guinea pig thalmic neurons and human cerebral cortex neurons. Therefore, it has been proposed that the molecular mechanism of volatile anesthetics involves an action on a specific class of K+ channels. The fact that a particular inhibitor synaptic K+ current, I_{K(An),} reversibly activated by volatile agents is present in anesthetic-sensitive molluscan pacemaker neurons, but absent in insensitive neurons has made it a very attractive candidate as a target for these important pharmacological agents. I_{K(As)} behaves as a background channel; it is not voltage-gated, it activates immediately, and it does not inactivate with time. I_{K(As)} obeys the Goldman-Hodgkin-Katz constant field equation and is resistant to the classical K+ channel blockers, tetraethylammonium and 4-aminopyridine.

Lopes et al « Action of general anaesthesic and arachidonci acid pathway inhibitors on K+ currents activated by volatile anaesthetics and FMRFamide in molluscan neurones » British journal of pharmacology (1998) 125, 309-318 discloses the study of K+ currents activated by volatile general anaesthetics. In particular, this document discloses the activation of background currents by volatile anaesthetic.

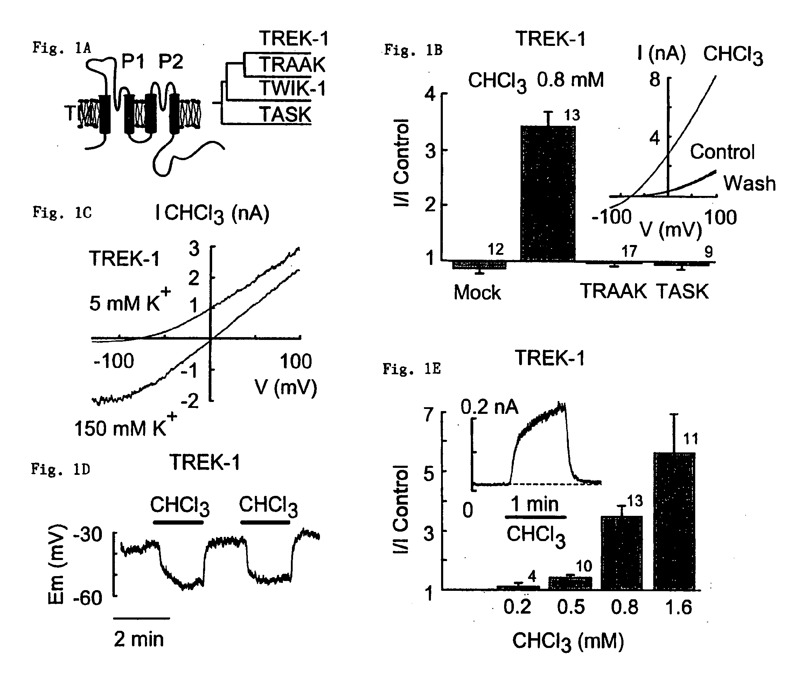

We recently identified a novel family of mammalian K+ channels with a unique structural motif consisting of two pore domains in tandem and four transmembrane segments. The four members in this family have been classified as TWIK-1, TASK, TREK-1, and TRAAK, which are shown in Fig. 1A. TWIK-1 has been previously shown to dimerize, implying that a functional channel is formed by at least two subunits. Heteromultimerization does not occur between the four members of this novel family as tested in Sf9 cells expressing various combinations of these channels (unpublished data). Three members of this family, mouse TREK-1, mouse TRAAK, and human TASK, encode for background outward-going K+ rectifiers with properties resembling those of I_{K(An)}. TRAAK and TREK-1 are directly opened by arachidonic acid and other polyunsaturated fatty acids, while TASK encodes a resting K+ channel which is controlled by external pH variations near physiological pH. TREK-1 and TASK are expressed in many tissues and are particularly abundant in the brain and in the heart, whereas TRAAK is selectively expressed in the central nervous system. Neuronal expression of these channels is detected at high levels in the cortex, cerebellum, hippocampus and olfactory bulb, and cardiac expression is detected in both the myocardium and connective tissues.

### BRIEF DESCRIPTION OF THE FIGURES

**Fig. 1** shows the results of experiments with chloroform demonstrating that chloroform selectively activates TREK-1: A, a schematic showing the two pore domains and four transmembrane domains of the 2P domain potassium K+ channels, TREK-1 TRAAK, TWIK-1 and TASK. The four transmembrane domain segments are indicated by TM1 through TM4, and the two pore regions are indicated by P1 and P2. The phylogenetic tree indicates the three subfamilies. TWIK-1 is an inward rectifier K+ channel and TASK is a background rectifier K+ channel inhibited by external acidosis. Both TREK-1 and TRAAK are background outward rectifier K+ channels opened by arachidonic acid; B, whole cell patch clamp experiments showing that 0.8 mM chloroform strongly and reversibly activates TREK-1 expressed in transfected COS cells. The mock condition is the wild-type (empty) expression vector. The effects of chloroform were investigated on K+ currents elicited in the whole cell configuration during a voltage ramp of one second in duration from a holding potential of -80 mV as illustrated in the inset for TREK-1; C, 0.8 mM chloroform induced a typical background current characterized by an outward-going rectification which reverses at the predicted value for E_{K+}; Chloroform activated TREK-1 currents in physiological and symmetrical K+ gradients were examined. Voltage ramps of one second in duration from a holding potential of -80 mV in both K+ conditions and in the presence of 0.8 mM chloroform were digitally subtracted from ramps in control conditions; D, 0.8 mM chloroform reversibly and reproducibly hyperpolarized COS cells expressing TREK-1; E, dose-dependence of TREK-1 activation. The inset illustrates the effect of 0.8 mM chloroform on TREK-1 current measured at a holding potential of 0 mV. The number of cells in each experimental condition is indicated.

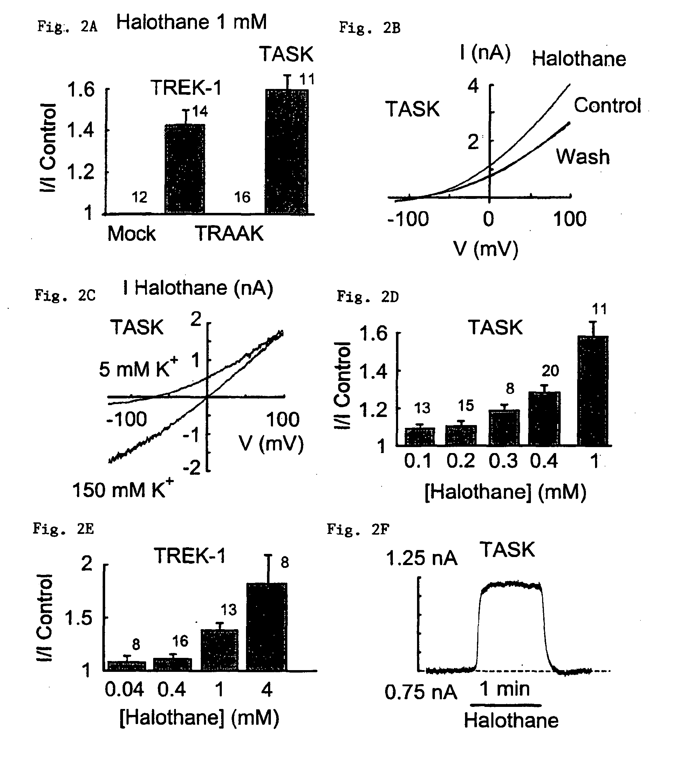

**Fig. 2** Halothane is a common activator of TREK-1 and TASK. A, comparative effects of 1 mM halothane on the 2P domain K+ channel activities. The mock condition is the wild-type (empty) expression vector; B, halothane (1 mM) stimulates TASK channel activity elicited in the whole cell configuration during voltage ramps of one second in duration from a holding potential of -80 mV; C, halothane (1 mM)-activated TASK currents in physiological and symmetrical K+ gradients. Voltage ramps of one second in duration from a holding potential of -80 mV in both K+ conditions and in the presence of 1 mM halothane are digitally subtracted from ramps in control conditions; D, dose-effect curve of halothane on TREK-1 channel activation; F, halothane (1 mM) reversibly activates TASK at a holding potential of 0 mV. The number of cells in each experimental condition is indicated.

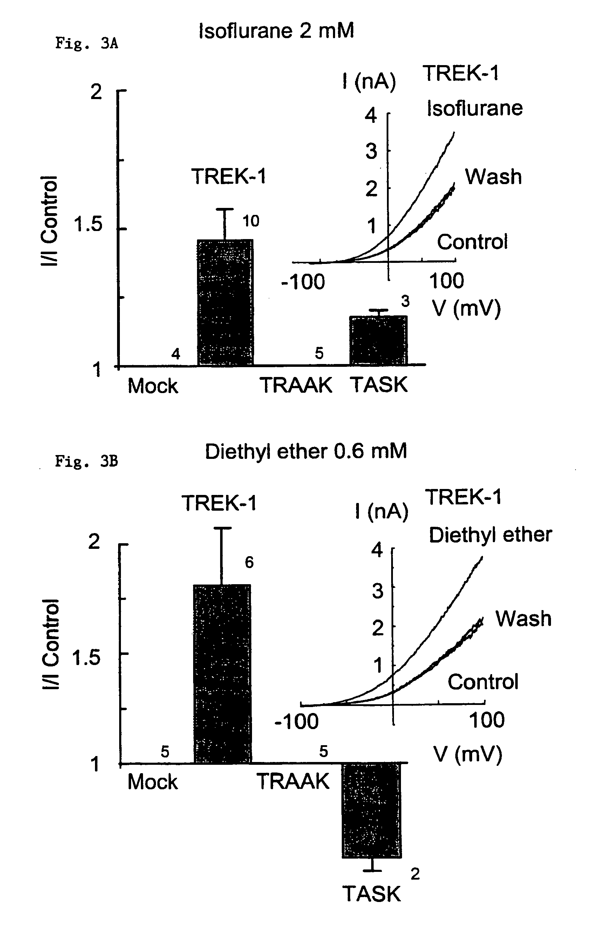

**Fig. 3** Isoflurane and diethyl ether differentially activate TREK-1 and TASK. A, comparative effects of 2 mM isoflurane (A) and 0.6 mM diethyl ether (B) on the 2P domain K+ channels. The mock condition is the wild-type (empty) expression vector. The effects of the anesthetics are investigated on K+ currents elicited in whole cell configuration during voltage ramps of one second in duration from a holding potential of -80 mV as illustrated in insets for TREK-1. The number of cells in each experimental condition is indicated.

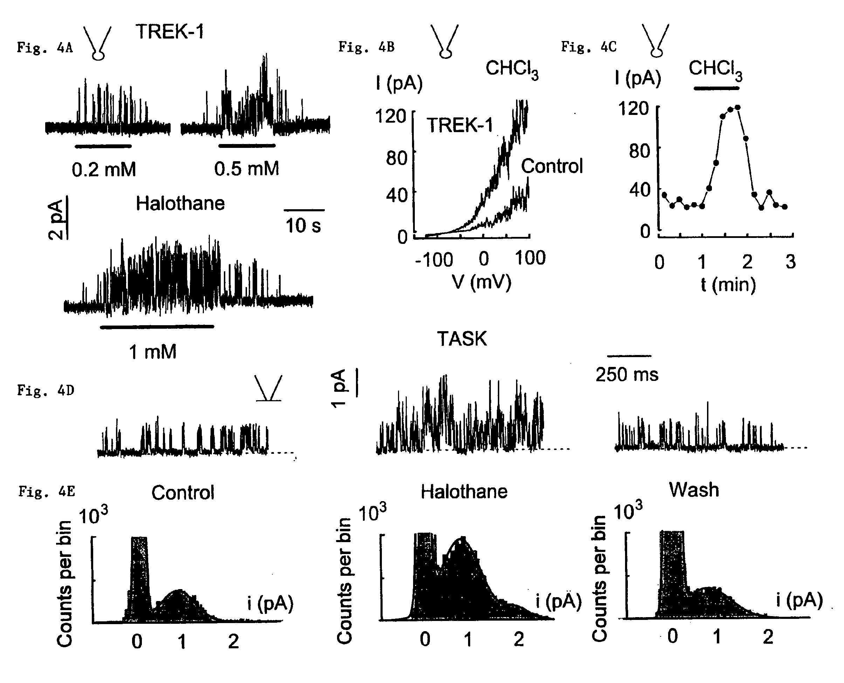

**Fig. 4** Volatile anesthetics stimulate TREK-1 and TASK in the excised patch configurations. A, effects of increasing concentrations of halothane on TREK-1 channel activity in an outside-out patch. In an outside-out patch, halothane reversibly opens a 48 pS TREK-1 channel in a dose-dependent fashion. The holding potential is 0 mV and applications of halothane are indicated by horizontal bars; B, effect of 0.8 mM chloroform on the I-V curve of TREK-1 in an outside-out patch. The I-V curve is performed with a voltage ramp of one second in duration from a holding potential of -80 mV; C, kinetics of activation of TREK-1 by 0.8 mM chloroform. The I-V curve of the chloroform-sensitive current in an outside-out patch shows the characteristic outward-going rectification; D, halothane (1 mM) induces TASK channel opening in an inside-out patch. The holding potential is 0 mV and channel activities before, during, and after addition of 1 mM halothane are illustrated from left to right. Halothane reversibly opens a 12 pS TASK channel.

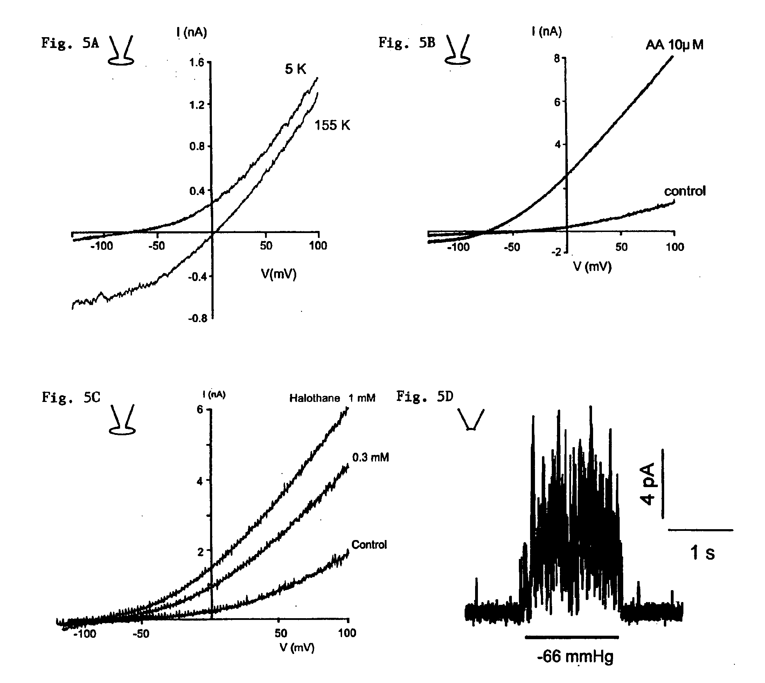

**Fig. 5** Functional characterization of the human TREK-1 (hTREK-1). Transiently transfected COS cells expressing hTREK-1 are voltage-clamped using the whole cell patch clamp configuration. A, The basal TREK-1 current is recorded in physiological K conditions (5 mM) and in symmetrical K conditions (155 mM). The cells are held at -80 mV and voltage ramps of 800 ms in duration are applied from -130 mV to 100 mV. B, hTREK-1 is stimulated by the addition of 10 µM arachidonic acid in the bath. Same protocol as in A. C, hTREK-1 is opened by increasing concentrations of halothane (as indicated). D, In the inside-out patch configuration hTREK-1 is opened by a membrane stretch of -66 mmHg. The patch is voltage clamped at 0 mV.

### SUMMARY OF THE INVENTION

### DETAILED DESCRIPTION OF THE INVENTION

An isolated nucleic acid molecule encoding human TREK-1 channel (SEQ ID NO:1) is disclosed the isolated human TREK-1 protein (SEQ ID NO:2) is also disclosed. The nucleic acid and deduced amino acid sequence is shown in SEQ ID NO:1.

The isolated nucleic acid molecule encoding the murine TREK-1 channel (SEQ ID NO:3) is disclosed. The isolated protein, murine TREK-1 (SEQ ID NO 4), is also disclosed.

The invention relates to a method for identifying substances having anesthetic properties upon inhalation comprising:
(a) contacting said substance with a mammalian potassium transport protein, wherein said potassium transport exhibits outward-going potassium rectification; and
(b) determining the potassium transport activity of said potassium transport protein, wherein an activation of potassium transport is indicative of said substance having said anesthetic properties,
wherein said mammalian potassium transport protein is TASK of sequence SEQ ID NO 5.

The method involves contacting a test substance with a potassium transport protein *in vitro* and determining whether there has been activation of potassium transport.

As used in the method, cells which express a potassium transport protein are used in the presence of the test substance. The test substance is a substance which will have certain properties when used in a mammal as a volatile inhalant. These properties may include the induction of a safe, reversible state of unconsciousness, amnesia and analgesia.

The cells expressing the potassium transport protein may is transiently express the protein. The cells may be selected from the group consisting of *Spodoptera, Xenophus* oocytes, Chinese hamster ovary cells, and fibroblast.

The potassium transport proteins which is suitable for use in the invention is TASK (SEQ ID NO:5), which may be derived from any mammalian source, such as rat, mouse, or human. The molecular sequence of TREE-1 may be the human TREK-1 as show in SEQ ID NO:2, or an amino acid sequence that is substantially identical to SEQ ID NOS. By "substantially identical" it is understood that amino acid substitutions may be made such that the overall conformation of the potassium transport protein is not significantly altered: the protein remains active as a potassium transport protein.

A suitable substantially identical protein is a protein having an amino acid sequence that is generally at least 90% identical to the amino acid sequence of human TREK-1 (SEQ ID NO:2). More preferably, the protein is at least 95% identical to SEQ ID NO:2. Most preferably, the amino acid sequence, is at least 99% identical to SEQ ID NO: 1.

The cells used in the method of the present invention express the potassium transport protein expressed on their surface transiently. Introduction of the nucleic acid into the cells so that the protein is expressed may be by any known method such as transfection of an appropriate nucleic acid construct into the cells, microinjection of RNA encoding the protein, and the like. Many different protocols are known in the art. Two methods are briefly described herein, however, it will be appreciated by one of ordinary skill in the art that many modifications and substitutions may be made to these methods without departing from the spirit and the scope of the invention.

The coding sequence of the potassium transport protein may be inserted between the noncoding sequences 5' and 3' of a *Xenopus laevis* protein (such as globin) in an appropriate vector, such as pEXO. The construct is introduced into an appropriate cell type to replicate the vector and/or to transcribe RNA. Alternatively, the vector may be used as a template for in vitro transcription. A complementary RNA (cRNA) is transcribed and injected into a cell, such as a *Xenopus* oocyte. Such a procedure may be performed in a 0.3 ml perfusion chamber, wherein single oocytes are impaled on two standard glass microelectrodes (0.5-2.0 MW) charged with 3 M KCl and maintained under voltage clamp with a Dagan TEV200 amplifier, The bath solution contains 98 mM KCl,1.8 mM CaCl₂, 2 mM MgCl₂, and 5 mM HEPES at pH 7.4 with KOH.

Alternatively, functional expression of the potassium channel may be accomplished by transfection of insect cells, such as *Spodoptera frugiperda* (Sf9) cells. Briefly a suitable vector, such as pVL1392 may be used and the coding sequence for the potassium transport protein may be inserted into the vector in-frame so that expression of the potassium transport protein may be expressed. The coding sequence for the potassium transport protein may be obtained by any convenient method, such as by PCR or by digesting a plasmid containing the potassium transport protein coding sequence with appropriate restriction endonuclease(s) for subsequent ligation into the pVL1392 vector. Similarly, the amplified product of the PCR may be digested with restriction enzymes and ligated into the vector. Transfection of SF9 cells may be performed by the manufacturer's protocol (Pharmingen).

Alternatively, functional expression of the potassium channel may be by transient transfection of cells such as COS cells whereby COS cells are seeded at a density of 20,000 cells per 35 mm dish. Cells are transfected with expression vector, such as the pIRES-CD8 vector, comprising the nucleic acid molecules encoding the desired potassium channel protein. The cells may be transfected by any method known in the art, such as the DEAE dextran protocol, Ca₂PO₄ precipitation, or electroporation, for example. The transfected cells, expressing the desired potassium channel, or induced to express the desired potassium channel may then be used in the method of the invention and the cells may be assayed for the transport of potassium.

The invention will be described in greater detail with reference to the examples which are provides to illustrate the invention. The examples are not to be construed to be limiting as to the scope of the invention, which is set forth in the appended claims.

### EXAMPLES

### 1. Cloning of the human TREK-1 channel

The TREK-1 channel was cloned by degenerate PCR technology using previously characterized murine sequence. Although such technology is extensively described in the literature and is familiar to those of ordinary skill in the art, briefly, degenerate oligonucleotide primers selected to amplify a region of murine TREK-1 were synthesized and placed in a polymerase chain reaction amplification using human DNA with appropriate buffer, nucleotides and DNA polymerase. The reaction is cycled through temperature stages for denaturation of DNA (generally about 94°C), annealing of primers to DNA template (this temperature can be varied to optimize amplification and can be based on many factors, including primer length and GC content), and extension of DNA polymerization by DNA polymerase (generally at the optimum temperature for the activity of the DNA polymerase which is usually about 72°C). The amplified DNA fragment may be isolated and cloned into a plasmid vector for subsequent sequence analysis, or the amplified DNA may be directly sequenced by known methods.

Due to the degeneracy of the DNA code, it will be well understood to one of ordinary skill in the art that substitution of nucleotides may be made without changing the amino acid sequence of the protein. Moreover, it is understood in the art that for a given protein's amino acid sequence, substitution of certain amino acids in the sequence can be made without significant effect on the function of the protein. Such substitutions are known in the art as "conservative substitutions." The encompasses human TREK I proteins that contain conservative substitutions, wherein the function of the protein is not altered is disclosed Generally, the identity of such an mutant TREK-1 will be at least 90% identical to SEQ ID NO:2. Preferably, the mutant TREK-1 will be at least 97 % identical to SEQ ID NO:2. Most preferably, the mutant TREK-1 will be at least 99 % identical to SEQ ID NO:2.

### 2. The sequencing of the murine TREK-1 channel

The sequence for murine TREK-1, which is a corrected form of murine TREK-1 reported earlier, is shown in SEQ ID NO:4. There is a longer open reading frame than originally reported, producing a protein with a deduced amino acid sequence of 411 amino acids. Due to the degeneracy of the DNA code, it will be well understood to one of ordinary skill in the art that substitution of nucleotides may be made without changing the amino acid sequence of the protein. Therefore, the invention includes any nucleic acid sequence for the murine TREK-1 channel that encodes the amino acid sequence determined for murine TREK-1 (SEQ ID NO:4). Moreover, as is the case with human TREK-1, it is understood in the art that for a given protein's amino acid sequence, substitution of certain amino acids in the sequence can be made without significant effect on the function of the protein. The invention encompasses murine TREK-1 proteins that contain conservative substitutions, wherein the function of the protein is not altered. Generally, the identity of such an mutant TREK-1 will be at least 90% identical to SEQ ID NO:4. Preferably, the mutant TREK-1 will be at least 95% identical to SEQ ID NO:4. More preferably, the mutant TREK-1 will be at least 97% identical to SEQ ID NO:4. Most preferably, the mutant TREK-1 will be at least 99% identical to SEQ ID NO:4.

### 3. Functional expression of the human TREK-1 channel

The functional properties of the human TREK-1 channel were studied on the basis of transfected COS cells temporarily expressing the protein. Like the murine channel, the human channel is selective for potassium and is activated by *cis*-unsaturated fatty acids, volatile anesthetics and by stretching of the plasma membrane. The selectivity of the TREK-1 currents for potassium is shown in Fig. 5A. The currents are recordings in the whole-cell configuration of the patch-clamp technique (imposed potential gradient from -130 to +100 mV). The inverse potential of the currents follow the equilibrium potential of potassium when the extracellular concentration of potassium ions climbs from 5 to 155 mM. Fig. 5B shows that the application of arachidonic acid (10 µM) induced activation of the TREK-1 currents. Fig. 5C shows that the application of volatile anesthetics (halothane in this case) at the concentrations employed in general anesthesia induced activation of the TREK-1 channel. Fig. 5D shows that the TREK-1 currents recorded in an excised patch (inside-out configuration) are mechanosensitive. When a pressure of -66 mmHg is applied on the membrane, the TREK-1 currents are activated in a reversible manner.

### 4. Electrophysiological recording

COS cell transfection, culture and electrophysiology are well known in the art and are described in the literature such as in the references cited.

As performed herein, COS cells were seeded at a density of 20,000 cells per 35 mm dish 24 hours prior to transfection. Cells were transfected by the DEAE dextran protocol (1 µg DNA per plate). Mouse TREK-1 (GenBank Accession No. U73488), human TASK (GenBank Accession No. AF006823) and mouse TRAAK (GenBank Accession No. AF056492) fragments were amplified by polymerase chain reaction (PCR) and subcloned into the pIRES-CD8 vector. Transfected cells were visualized 48 hours after transfection using the anti-CD8 antibody coated beads method. For whole cell and excised patch experiments, the internal solution was 150 mM KCl, 3 mM MgCl₂, 5mM EGTA and 10 mM HEPES at pH 7.2 with KOH and the external medium contained 150 mM NaCl, 5 mM KCL, 3 mM MgCl₂, 1 mM CaCl₂, 10 mM HEPES at pH 7.4 with NaOH. Cells were continuously superfused with a microperfusion system during the time course of the experiments (0.1 ml/min) performed at room temperature. A RK300 patch clamp amplifier (Biologic, Grenoble, France) was used for whole cell as well as single channel recordings. Ionic currents were monitored and recorded continuously using a DAT recorder (Biologic, Grenoble, France). Subsequently, data were replayed and sampled using pClamp software. Data analysis were performed using clampfit (pClamp) for whole cell recording as well as Biopatch (Biologic) software for single channel recordings. Membrane capacitance was measured during a 10 V hyperpolarizing step from a holding potential of -80 mV. Student's t test was used for statistical analysis (P<0.001).

### 5. Anesthetics delivery and concentration

General anesthetics were directly dissolved in saline solutions. All experiments were performed at room temperature (24°C). Solutions were prepared every 3 hours in gas-tight bottles as stock solutions (calculated concentrations of 5 10 mM). Serial dilutions were prepared just prior to the electrophysiological experiment. 2.5 ml of each experimental solution at the desired concentration was placed in a syringe connected to the experimental superfusion system. The electrophysiological measurements were performed within 45 minutes.

The actual concentrations of anesthetics were subsequently determined by means of a gas chromatography method (HP 6890 equipped with a DB624 column) using FID detection. Sampled (2.5 ml of solution) were collected prior to (t₀) and after perfusion (t₄₅) through the experimental setup. Solutions were collected using gas impermeable tubing and stored in sealed glass containers at 4°C for subsequent analysis. Samplings and measurements were performed in duplicate. Actual concentrations of anesthetics were determined by multiplying the calculated concentration by the ratio t₄₅/t₀ (chloroform: 0.16; halothane: 0.37; isoflurane: 0.76; and diethyl ether: 0.57). In the dose effect curves, the threshold concentrations were estimated as concentrations producing an increase higher than 10% in current amplitude.

### RESULTS

The nucleic acid sequence corresponding to the open reading frame of the human TREK-1 channel is shown in SEQ ID NO:1. The sequence of 1236 nucleotides encodes a protein of 411 amino acids. The conservation between human and murine proteins is very high, exceeding 99% homology.

TASK and TREK-1, two mammalian 2P domain K+ channels which have similar properties to I_{K(An)} are activated by volatile general anesthetics. Chloroform, diethyl ether, halothane and isoflurane activated TREK-1, while only halothane and isoflurane activated TASK. The C-terminal regions of both TREK-1 and TASK are critical for anesthetic activation. Thus, both TREK-1 and TASK are important target sites for these agents.

In whole patch clamp experiments shown in Fig. 1B, chloroform strongly and reversibly activated TREK-1 expressed in transfected COS cells, while it slightly depressed TASK and did not effect TRAAK. As shown in Fig. 1C, chloroform induced a typical background current, characterized by an outward-going rectification which reverses at the predicted value for E_{K}⁺ as demonstrated by the shift of the reversal potential to 0 mV in symmetrical K⁺ conditions. No current activation was observed in mock-transfected cells (Fig. 1B). Fig. 1D shows that chloroform also reversibly and reproducibly hyperpolarized COS cells expressing TREK-1 (but not mock-transfected cells, not shown; n = 5). TREK-1 activation was dose dependent, with a threshold concentration for activation of 500 µM, as shown in Fig. 1E. The inset of Fig. 1E shows that current activation, measured at a holding potential of 0 mV, started rapidly, but steady-state activation was barely reached after one minute in the presence of chloroform.

Both TREK and TASK, but not TRAAK, were opened by halothane. As shown in Fig. 2B and 2C, halothane-induced current displayed an outward-going rectification and revered at the predicted value for E_{K}⁺. The dose-effect curves (shown in Fig. 2D and Fig. 2E) demonstrated that the threshold concentrations for halothane on TREK-1 and TASK are 400 µM and 200 µM, respectively. The effects of halothane on TASK were rapid completely reversible. Opening of TASK was faster compared to the opening of TREK-1 by chloroform (compare Fig. 2F with Fig. 1E, inset). These results suggest that the components of the activation of both channels by anesthetics may be mediated by different molecular mechanisms.

Isoflurane, like halothane, activated both TREK-1 and TASK channels without altering TRAAK, as shown in Fig. 3A. Diethyl ether, like chloroform, opened TREK-1, but did not affect TRAAK. Diethyl ether also decreased TASK activity.

To demonstrate that activation by volatile anesthetics does not occur via second messenger pathways, experiments were conducted on excised patches. Fig. 4A shows that in an excised outside-out patch, halothane reversibly, and in a dose dependent manner, opened a 48 pS TREK-1 channel. No channel activity was observed in the absence of the anesthetic, suggesting that halothane converts inactive channels into active channels. The I-V curve of the chloroform-sensitive current in an outside-out patch shows the characteristic outward-going rectification previously observed in the macroscopic whole cell conditions, as shown in Figs. 4B-4C and Fig. 1B (inset). As shown in Figs. 4D-4E, in the inside-out patch configuration, halothane reversibly opened a 12 pS TASK channel. In the absence of anesthetics, a single TASK channel was opened, as shown in the left panels of Figs. 4D-4E. The addition of halothane induced the opening of a second channel (see Figs. 4D-4E middle panel) which closed again after washing (see right panel of Figs. 4D and 4E). All the data, taken together, demonstrate that volatile general anesthetics open TASK and TREK-1 channels and that these effects are likely to be direct and independent of second messengers.

TREK-1 and TASK are probably critical channels for the action of general volatile anesthetics. Opening of these K+ channels along with the known modulation of neurotransmitter receptors will probably explain general anesthetic action. Effects of volatile anesthetics on ligand-gated ion channels such as GABA_{A} receptors are probably particularly important at the post-synaptic level. Opening of background TREK-1 and TASK channels by volatile anesthetics might be very important both at the pre-synaptic (I_{K(An)}) and at the post-synaptic level. An activation of only a small fraction of these I_{K(An)} channels will be expected to have significant effects on membrane polarization and consequently potentially important effects in both pre- and post-synaptic functions. TREK-1 and TASK channels are expressed in neuronal cells but they are also expressed in other tissues, and particularly in the heart. It is, therefore, not surprising that volatile anesthetics have depressive side-effects on heart function. These effects include a slowing of the heart rate and negative inotropic effects and are fully compatible with an exaggerate opening of background K+ channels by volatile anesthetics. The same considerations might very well explain ventilatory depression.

## Claims

1. A method for identifying substances having anesthetic properties upon inhalation comprising:
(a) contacting said substance with a mammalian potassium transport protein, wherein said potassium transport exhibits outward-going potassium rectification; and
(b) determining the potassium transport activity of said potassium transport protein, wherein an activation of potassium transport is indicative of said substance having said anesthetic properties,
wherein said mammalian potassium transport protein is TASK of sequence SEQ ID NO 5.

2. The method of claim 1 comprising:
(a) contacting said substance with transfected cells, wherein said transfected cells are transfected with a nucleotide vector comprising a nucleic acid molecule encoding said TASK, wherein said transfected cells transiently express said TASK on a surface of said transfected cells, and wherein said TASK exhibits outward-going potassium rectification; and
(b) determining the potassium transport activity of said TASK, wherein an activation of potassium transport is indicative of said substance having said anesthetic properties.

3. The method of claim 2, wherein said transfected cells are selected from the group consisting of *Spodoptera, Xenopus* oocytes, Chinese hamster ovary cells, and fibroblasts.

## Patentansprüche

1. Ein Verfahren zur Identifizierung von Substanzen mit anästhesierenden Eigenschaften durch Inhalation, das folgende Schritte umfasst :
a. Die besagte Substanz wird mit einem Kaliumtransportprotein eines Säugetiers in Kontakt gebracht, wobei besagter Kaliumtransport eine auswärtsgerichtete Kaliumberichtigung aufzeigt und
b. die Kaliumtransportaktivität des besagten Kaliumtransportproteins wird bestimmt, wobei eine Aktivierung des Kaliumtransports anzeigt, dass besagte Substanz besagte anästhesierenden Eigenschaften besitzt,
wobei besagtes Kaliumtransportprotein eines Säugetiers TASK Sequenz SEQ ID NO 5 ist.

2. Verfahren gemäß Anspruch 1, das folgende Schritte umfasst :
a. besagte Substanz wird mit transfektierten Zellen in Kontakt gebracht, wobei besagte transfektierte Zellen mit einem Nukleotidvektor transfektiert sind, der ein Nukleinsäuremolekül enthält, das besagtes TASK kodiert, wobei besagte transfektierte Zellen vorübergehend besagtes TASK auf einer Oberfläche der besagten transfektierten Zellen ausdrücken, und wobei besagtes TASK eine auswärts gerichtete Kaliumberichtigung aufweist; und
b. die Kaliumtransportaktivität des besagten TASK bestimmt wird, wobei eine Aktivierung des Kaliumtransports anzeigt, dass besagte Substanz besagte anästhetischen Eigenschaften besitzt,

3. Verfahren gemäß Anspruch 2, wobei besagte transfektierte Zellen innerhalb der Gruppe gewählt werden, die aus *Spodoptera, Xenopus*-Ovozyten, Ovarzellen des chinesischen Hamsters und Fibroblasten besteht.

## Revendications

1. Un procédé pour identifier des substances ayant des propriétés anesthésiques par inhalation comprenant :
a. la mise en contact de ladite substance avec une protéine de mammifère de transport du potassium, dans lequel ledit transport de potassium présente une rectification potassique sortante ; et
b. la détermination de l'activité du transport potassique de ladite protéine de transport de potassium, dans lequel une activation du transport potassique indique que ladite substance a lesdites propriétés anesthésiques,
dans lequel ladite protéine de mammifère de transport de potassium est TASK de séquence SEQ ID NO 5.

2. Procédé selon la revendication 1 comprenant
a. la mise en contact de ladite substance avec des cellules transfectées, dans lequel lesdites cellules transfectées sont transfectées avec un vecteur nucléotidique comprenant une molécule d'acide nucléique codant ladite TASK, dans lequel lesdites cellules transfectées expriment transitoirement ladite TASK sur la surface desdites cellules transfectées, et dans lequel ladite TASK présente une rectification potassique sortante, et
b. la détermination de l'activité de transport potassique de ladite TASK, dans lequel une activation du transport potassique indique que ladite substance a lesdites propriétés anesthésiques.

3. Procédé selon la revendication 2, dans lequel lesdites cellules transfectées sont choisies dans le groupe constitué de *Spodoptera,* d'ovocytes de *Xenopus,* de cellules ovariennes d'hamster chinois, et de fibroblastes.


### SEQUENCE LISTING

<110> PATEL, AMANDA J.
   HONORE, ERIC
   LESAGE, FLORIAN
   ROMEY, GEORGES
   LAZDUSKI, MICHEL
<120> A method for the identification of anesthetics
<130> f17b12prov3-humanTREK
<140> 6079727
   <141> 1999-02-12
<160> 4
<170> Wordperfect 8.0
<210> 1
   <211> 1236
   <212> ADN
   <213> Homo sapiens
<220>
   <221> CDS
   <222> (1) .. (1236)
<400> 1
   atg gcg gcc cct gac ttg ctg gat cct aaa tct gcc gct cag aac tcc      48
   aaa ccg agg ctc tcg ttt tcc acg aaa ccc aca gtg ctt gct tcc cgg      96
   gtg gag agt gac acg acc att aat gtt atg aaa tgg aag acg gtc tcc     144
   acg ata ttc ctg gtg gtt gtc ctc tat ctg atc atc gga gcc acc gtg     192
   ttc aaa gca ttg gag cag cct cat gag att tca cag agg acc acc att     240
   gtg atc cag aag caa aca ttc ata tcc caa cat tcc tgt gtc aat tcg     288
   acg gag ctg gat gaa ctc att cag caa ata gtg gca gca ata aat gca     336
   ggg att ata ccg tta gga aac acc tcc aat caa atc agt cac tgg gat     384
   ttg gga agt tcc ttc ttc ttt gct ggc act gtt att aca acc ata gga     432
   ttt gga aac atc tca cca cgc aca gaa ggc ggc aaa ata ttc tgt atc    480
   atc tat gcc tta ctg gga att ccc ctc ttt ggt ttc ctc ttg gct gga    528
   gtt gga gat cag cta ggc acc ata ttt gga aaa gga att gcc aaa gtg    576
   gaa gat acg ttt att aag tgg aat gtt agt cag acc aag att cgc atc    624
   atc tca aca atc ata ttt ata cta ttt ggc tgt gta ctc ttt gtg gct    672
   ctg cct gcg atc ata ttc aaa cac ata gaa ggc tgg agt gcc ctg gac    720
   gcc att tat ttt gtg gtt atc act cta aca act att gga ttt ggt gac    768
   tac gtt gca ggt gga tcc gat att gaa tat ctg gac ttc tat aag cct    816
   gtc gtg tgg ttc tgg atc ctt gta ggg ctt gct tac ttt gct gct gtc    864
   ctg agc atg att gga gat tgg ctc cga gtg ata tct aaa aag aca aaa    912
   gaa gag gtg gga gag ttc aga gca cac gct gct gag tgg aca gcc aac    960
   gtc aca gcc gaa ttc aaa gaa acc agg agg cga ctg agt gtg gag att   1008
   tat gac aag ttc cag cgg gcc acc tcc atc aag cgg aag ctc tcg gca   1056
   gaa ctg gct gga aac cac aat cag gag ctg act cct tgt agg agg acc   1104
   ctg tca gtg aac cac ctg acc aac gag agg gat gtc ttg cct ccc tta   1152
   ctg aag act gag agt atc tat ctg aat ggt ttg acg cca cac tgt gct   1200
   ggt gaa gag att gct gtg att gag aac atc aaa tag                1236
<210> 2
   <211> 411
   <212> PRT
   <213> Homo sapiens
<400> 2
<210> 3
   <211> 3580
   <212> ADN
   <213> Mus musculus
<220>
   <221> CDS
   <222> (484) .. (1719)
<400> 3
   agagcggcga ggcgagggga gagtggtgct acgggccagg cgggccaccc cgggccacac 60
   ccccaccttg cgggcgcccg gcggggctcg agccaggcgg ggcgcctcac aaagacatgc 120
   gaagaggggc tgcagtgatc accccctcgc tgagccccgg ggcagagccc agccgccggc 180
   cgagcgcacg gagccacggg ccgagcgcac ccagggcccg cgcgggaccc caggcggcca 240
   cgcaatcggg gtgacccatc gcgcgcgggg gcgtcgtcgt ccgatcccaa cttggcctcg 300
   gcctcgccct ctgcccagcc tgccaccgct ggtgtcctct ccttccggcg atttcgtttc 360
   ttctcacgct cccccctcta tacccctccc gcctccagcc ccgctctccc caccttgtaa 420
   aacaaagccg gggaaaatgc ctacccgtgc agctcggagc gcgcagcccg tcttggaata 480
   agg atg gcg gcc cct gac ttg ctg gat ccc aag tct gct gct cag aac     528
       Met Ala Ala Pro Asp Leu Leu Asp Pro Lys Ser Ala Ala Gln Asn
       1               5                   10                  15
   tcc aaa ccg agg ctc tca ttc tcc tca aaa ccc acc gtg ctt gct tcc     576
   Ser Lys Pro Arg Leu Ser Phe Ser Ser Lys Pro Thr Val Leu Ala Ser
                   20                  25                  30
   cgg gtg gag agt gac tcg gcc att aat gtt atg aaa tgg aag aca gtc     624
   Arg Val Glu Ser Asp Ser Ala Ile Asn Val Met Lys Trp Lys Thr Val
               35                  40                  45
   tcc acg att ttc ctg gtg gtc gtc ctc tac ctg atc atc gga gcc gcg     672
   Ser Thr Ile Phe Leu Val Val Val Leu Tyr Leu Ile Ile Gly Ala Ala
           50                  55                  60
   gtg ttc aag gca ttg gag cag cct cag gag att tcc cag agg acc acc     720
   Val Phe Lys Ala Leu Glu Gln Pro Gln Glu Ile Ser Gln Arg Thr Thr
       65                  70                  75
   att gtg atc cag aag cag acc ttc ata gcc cag cat gcc tgc gtc aac     768
   Ile Val Ile Gln Lys Gln Thr Phe Ile Ala Gln His Ala Cys Val Asn
   80                  85                  90                  95
   tcc acc gag ctg gac gaa ctc atc cag caa ata gtg gca gca ata aac     816
   Ser Thr Glu Leu Asp Glu Leu Ile Gln Gln Ile Val Ala Ala Ile Asn
                   100                 105                 110
   gca ggg att atc ccc tta gga aac agc tcc aat caa gtt agt cac tgg     864
   Ala Gly Ile Ile Pro Leu Gly Asn Ser Ser Asn Gln Val Ser His Trp
               115                 120                 125
   gac ctc gga agc tct ttc ttc ttt gct ggt act gtt atc aca acc ata     912
   Asp Leu Gly Ser Ser Phe Phe Phe Ala Gly Thr Val Ile Thr Thr Ile
           130                 135                 140
   gga ttt gga aac atc tcc cca cga act gaa ggt gga aaa ata ttc tgc     960
   Gly Phe Gly Asn Ile Ser Pro Arg Thr Glu Gly Gly Lys Ile Phe Cys
       145                 150                 155
   atc atc tat gcc ttg ctg gga att ccc ctc ttt ggc ttt cta ctg gct    1008
   Ile Ile Tyr Ala Leu Leu Gly Ile Pro Leu Phe Gly Phe Leu Leu Ala
   160                 165                 170                 175
   ggg gtt ggt gat cag cta gga act ata ttt gga aaa gga att gcc aaa    1056
   Gly Val Gly Asp Gln Leu Gly Thr Ile Phe Gly Lys Gly Ile Ala Lys
                   180                 185                 190
   gtg gaa gac aca ttt att aag tgg aat gtt agt cag acg aag att cgt    1104
   Val Glu Asp Thr Phe Ile Lys Trp Asn Val Ser Gln Thr Lys Ile Arg
               195                 200                 205
   atc atc tcc acc atc atc ttc atc ctg ttt ggc tgt gtc ctc ttt gtg    1152
   Ile Ile Ser Thr Ile Ile Phe Ile Leu Phe Gly Cys Val Leu Phe Val
           210                 215                 220
   gct ctc cct gcg gtc ata ttc aag cac ata gaa ggc tgg agc gcc ctg    1200
   Ala Leu Pro Ala Val Ile Phe Lys His Ile Glu Gly Trp Ser Ala Leu
       225                 230                 235
   gac gct atc tat ttt gtg gtt atc act ctg acg acc att gga ttt gga    1248
   Asp Ala Ile Tyr Phe Val Val Ile Thr Leu Thr Thr Ile Gly Phe Gly
   240                 245                 250                 255
   gac tac gtg gca ggt gga tca gac att gaa tat ctg gac ttc tac aag    1296
   Asp Tyr Val Ala Gly Gly Ser Asp Ile Glu Tyr Leu Asp Phe Tyr Lys
                   260                 265                 270
   cct gtg gtg tgg ttc tgg atc ctc gtt ggg ctg gcc tac ttt gca gct    1344
   Pro Val Val Trp Phe Trp Ile Leu Val Gly Leu Ala Tyr Phe Ala Ala
               275                 280                 285
   gtt ctg agc atg att ggg gac tgg cta cgg gtg atc tct aag aag acg    1392
   Val Leu Ser Met Ile Gly Asp Trp Leu Arg Val Ile Ser Lys Lys Thr
           290                 295                 300
   aag gaa gag gtg gga gag ttc aga gcg cat gcc gct gag tgg aca gcc    1440
   Lys Glu Glu Val Gly Glu Phe Arg Ala His Ala Ala Glu Trp Thr Ala
       305                 310                 315
   aat gtc acg gcc gag ttc aag gaa acg agg agg cgg ctg agc gtg gag    1488
   Asn Val Thr Ala Glu Phe Lys Glu Thr Arg Arg Arg Leu Ser Val Glu
   320                 325                 330                 335
   atc tac gac aag ttc cag cgt gcc aca tcc gtg aag cgg aag ctc tcc    1536
   Ile Tyr Asp Lys Phe Gln Arg Ala Thr Ser Val Lys Arg Lys Leu Ser
                   340                 345                 350
   gca gag ctg gcg ggc aac cac aac cag gaa ctg act ccg tgt agg agg    1584
   Ala Glu Leu Ala Gly Asn His Asn Gln Glu Leu Thr Pro Cys Arg Arg
               355                 360                 365
   acc ctg tct gtg aac cac ctg acc agc gag agg gaa gtc ctg cct ccc    1632
   Thr Leu Ser Val Asn His Leu Thr Ser Glu Arg Glu Val Leu Pro Pro
           370                 375                 380
   ttg ctg aag gct gag agc atc tat ctg aac ggt ctg aca cca cac tgt    1680
   Leu Leu Lys Ala Glu Ser Ile Tyr Leu Asn Gly Leu Thr Pro His Cys
       385                 390                 395
   gct ggt gag gac ata gct gtc att gag aac atg aag tag ccctctcttg    1729
   Ala Gly Glu Asp Ile Ala Val Ile Glu Asn Met Lys
   400 405 410
   gaagagtctg aggtggagcc atagggaagg gcttctctag gctctttgtg actgttgccg 1789
   gtagcattta aacattgtgc atggtgacct caaagggaaa gcaaatagaa aacacccatc 1849
   tggtcacctt acatccaggg agggtgttgt cccgaggcgg cactctgagg atgccgtgtg 1909
   ctgtccgctg agtgctgagt gatggacagg cagtgtctga tgccttttgt gcccagactg 1969
   tttcccctcc ccctctctcc taacgtgcca taaggcctat gaatgaatct gaatgctttg 2029
   ctggtcatgt agattggagg gatcagccct tggtttttca tggttcacct aactgagcct 2089
   ggatactgac cacttaggga tgacaacatt tctttttgta aatggcgaga aattcttacg 2149
   cagcctttta cctaagaaat tttctgccag tgccttatct tatgaagaaa caagaccctg 2209
   tctggtgggc ttgtggttcc tcccttcccc gccctcaccg tgggctcacc ctttgctgac 2269
   tctcacctat cccctcccct ccgcatacct tattgtgctg gaagcaatgt gtggcttgat 2329
   ggaagaaaca gatgccaact gcaggcacag aagcaacatg gaagctctgg cgtcacgggc 2389
   actgcagaga agggaggtca gagaaggccc ctctgagtat ttatttgacc ggggtaccaa 2449
   tggtacagct atatatatgt acagagtaat aattcccagg ccggtaacct tggctgcttc 2509
   cacacggccc ccttttttcc ctggcagtat ttgaagttta tcatttatta ataactagtc 2569
   atttttaaag gcagaagaag acatgagcac atacatctgt aatctacgtg atgtgataag 2629
   aaaactgttc agactggtat tgcaaggtga tctcagacag tcgatatgga ttcattctga 2689
   tgagaagaaa acagcgagac cacgtgtcgt gcgcatgagc tcatacaggg ctcactgtca 2749
   gcttggacca gcccgtgagg tacagcaggc ttacacactt tcttttctta agtggttctt 2809
   gccaaactgg agggaggggc gatgcccttc agaagggggc acagccccag ccagcccagg 2869
   gtccctctgt ggtcatgact gggtgtgagc acagatgctg gccttgggat cactgtgagt 2929
   tttgcacatg gagagataca gactgctggc ataggtcgtc tctaacagta gagaaaacgc 2989
   cgattagcac aatctaaatc ccccgagtag ctttttgttt aggataagag aaggctggta 3049
   attcacttaa tttaaattta tatcctataa ttctttttgg atgtttcaag attcagaaaa 3109
   agtccagtcc ctgcatctag caaaccgccg ccctttcctc tgtgcccgta cttacatcta 3169
   ctgaacactg tatatgtaat ttttaaattt ttaaagcgca gaaggaaaat gattcttcta 3229
   catgtaatcg caaaactgat ttctcccttc tgggggaggc ttgggcttac gtgatcatgt 3289
   ggcattcaga gtaaagtctt aagacataaa cccaggatgt ttaaaaacac aaaaagattc 3349
   ctattttcca aaatttgaat ttaagctata aatgtaaata tatccagttc gcttgcagag 3409
   agtactttta taaacctatg aagattctaa ccaaaatttt aaaatgtcgg gttcctttac 3469
   aaagaaagct ccacccatct cctgaatagc cgttttggaa gctgacatgg tgggatgtac 3529
   cacgtataaa ctgtgaactg gaggacaaat aaagtttgta attaaaaaaa a 3580
<210> 4
   <211> 411
   <212> PRT
   <213> Mus musculus
<400> 4
   Met Ala Ala Pro Asp Leu Leu Asp Pro Lys Ser Ala Ala Gln Asn Ser
   1 5 10 15
   Lys Pro Arg Leu Ser Phe Ser Ser Lys Pro Thr Val Leu Ala Ser Arg
   20 25 30
   Val Glu Ser Asp Ser Ala Ile Asn Val Met Lys Trp Lys Thr Val Ser
   35 40 45
   Thr Ile Phe Leu Val Val Val Leu Tyr Leu Ile Ile Gly Ala Ala Val
   50 55 60
   Phe Lys Ala Leu Glu Gln Pro Gln Glu Ile Ser Gln Arg Thr Thr Ile
   65 70 75 80
   Val Ile Gln Lys Gln Thr Phe Ile Ala Gln His Ala Cys Val Asn Ser
   85 90 95
   Thr Glu Leu Asp Glu Leu Ile Gln Gln Ile Val Ala Ala Ile Asn Ala
   100 105 110
   Gly Ile Ile Pro Leu Gly Asn Ser Ser Asn Gln Val Ser His Trp Asp
   115 120 125
   Leu Gly Ser Ser Phe Phe Phe Ala Gly Thr Val Ile Thr Thr Ile Gly
   130 135 140
   Phe Gly Asn Ile Ser Pro Arg Thr Glu Gly Gly Lys Ile Phe Cys Ile
   145 150 155 160
   Ile Tyr Ala Leu Leu Gly Ile Pro Leu Phe Gly Phe Leu Leu Ala Gly
   165 170 175
   Val Gly Asp Gln Leu Gly Thr Ile Phe Gly Lys Gly Ile Ala Lys Val
   180 185 190
   Glu Asp Thr Phe Ile Lys Trp Asn Val Ser Gln Thr Lys Ile Arg Ile
   195 200 205
   Ile Ser Thr Ile Ile Phe Ile Leu Phe Gly Cys Val Leu Phe Val Ala
   210 215 220
   Leu Pro Ala Val Ile Phe Lys His Ile Glu Gly Trp Ser Ala Leu Asp
   225 230 235 240
   Ala Ile Tyr Phe Val Val Ile Thr Leu Thr Thr Ile Gly Phe Gly Asp
   245 250 255
   Tyr Val Ala Gly Gly Ser Asp Ile Glu Tyr Leu Asp Phe Tyr Lys Pro
   260 265 270
   Val Val Trp Phe Trp Ile Leu Val Gly Leu Ala Tyr Phe Ala Ala Val
   275 280 285
   Leu Ser Met Ile Gly Asp Trp Leu Arg Val Ile Ser Lys Lys Thr Lys
   290 295 300
   Glu Glu Val Gly Glu Phe Arg Ala His Ala Ala Glu Trp Thr Ala Asn
   305 310 315 320
   Val Thr Ala Glu Phe Lys Glu Thr Arg Arg Arg Leu Ser Val Glu Ile
   325 330 335
   Tyr Asp Lys Phe Gln Arg Ala Thr Ser Val Lys Arg Lys Leu Ser Ala
   340 345 350
   Glu Leu Ala Gly Asn His Asn Gln Glu Leu Thr Pro Cys Arg Arg Thr
   355 360 365
   Leu Ser Val Asn His Leu Thr Ser Glu Arg Glu Val Leu Pro Pro Leu
   370 375 380
   Leu Lys Ala Glu Ser Ile Tyr Leu Asn Gly Leu Thr Pro His Cys Ala
   385 390 395 400
   Gly Glu Asp Ile Ala Val Ile Glu Asn Met Lys
   405 410
<210> 5
   <211> 394
   <212> PRT
   <213> Mus sp.
   <223> TASK
<400> 5